# FASCICULE DE BREVET EUROPEEN

(11) **EP 2 326 708 B1**
(45) Date de publication et mention de la délivrance du brevet: **23.03.2016**
(21) Numéro de dépôt: 09737040.7
(22) Date de dépôt: 28.08.2009
(51) Int. Cl.: C12N 1/12, A61K 8/99, A61K 8/58, A61K 36/02, A23L 33/10

(54) **MICROORQANISMES PHOTOSYNTHÉTIQUES ENRICHIS EN SÉLÉNIUM À PARTIR DE COMPOSÉS SÉLÉNO-HYDROXYACIDES, LEURS APPLICATIONS EN NUTRITION, COSMÉTIQUE ET PHARMACIE**
AN SELEN ANGEREICHERTE PHOTOSYNTHETISCHE MIKROORGANISMEN UNTER VERWENDUNG VON SELENOHYDROXYSÄUREVERBINDUNGEN, VERWENDUNGEN DAVON BEI ERNÄHRUNG, KOSMETIK UND PHARMAZIE
PHOTOSYNTHETIC MICROORGANISMS ENRICHED IN SELENIUM USING SELENOHYDROXY ACID COMPOUNDS, USES THEREOF IN NUTRITION, COSMETICS AND PHARMACY

(30) Priorité: 29.08.2008 FR 0855827
(43) Date de publication de la demande: 01.06.2011
(73) Titulaire: Metabolium, 93230 Romainville (FR)
(72) Inventeur: KUDLA, Bernard, F-91470 Les Molieres (FR); DE BAENE, Frédéric, F-77500 Chelles (FR); LANGE, Marc, F-75014 Paris (FR)
(74) Mandataire: Santarelli
(86) Numéro de dépôt international: PCT/FR2009/001044
(87) Numéro de publication internationale: WO 2010/023384

(56) Documents cités:
- WO-A-2006/008190
- GB-A- 2 214 928
- US-A1- 2007 053 866
- CASES JULIEN ET AL: "Selenium from selenium-rich spirulina is less bioavailable than selenium from sodium selenite and selenomethionine in selenium-deficient rats" JOURNAL OF NUTRITION, vol. 131, no. 9, septembre 2001 (2001-09), pages 2343-2350, XP002526985 ISSN: 0022-3166
- LARSEN E H ET AL: "Speciation of selenoamino acids, selenonium ions and inorganic selenium by ion exchange HPLC with mass spectrometric detection and its application to yeast and algae" JOURNAL OF ANALYTICAL ATOMIC SPECTROMETRY DECEMBER 2001 ROYAL SOCIETY OF CHEMISTRY GB, vol. 16, no. 12, décembre 2001 (2001-12), pages 1403-1408, XP002526986
- DE SOUZA MARK P ET AL: "Selenium assimilation and volatilization from dimethylselenoniopropionate by Indian mustard" PLANT PHYSIOLOGY (ROCKVILLE), vol. 122, no. 4, avril 2000 (2000-04), pages 1281-1288, XP002526987 ISSN: 0032-0889
- Douskova I. et al.: "Scenedesmus quadricauda - a promising microorganism for selenium-enriched algal biomass production", Poster at Symposium for European Freshwater Sciences SEFS-5, Palermo 8-13 July 2007 , 10 July 2007 (2007-07-10), XP55016178, Retrieved from the Internet: URL:http://www.effsonline.org/index/sefs/s efs5/papers/contentParagraph/0114/document /Douskova.pdf [retrieved on 2012-01-11]
- BESSER JOHN M ET AL: "Bioaccumulation of organic and inorganic selenium in a laboratory food", ENVIRONMENTAL TOXICOLOGY AND CHEMISTRY, PERGAMON PRESS, US, vol. 12, no. 1, 1 January 1993 (1993-01-01), pages 57-72, XP008155489, ISSN: 0730-7268
- P. M. Neumann ET AL: "Rapid microalgal metabolism of selenate to volatile dimethylselenide", Plant, Cell & Environment, vol. 26, no. 6, 1 June 2003 (2003-06-01), pages 897-905, XP055128844, ISSN: 0140-7791, DOI: 10.1046/j.1365-3040.2003.01022.x

## Description

L'invention se rapporte à l'enrichissement de microorganismes photosynthétiques en sélénium organique, notamment à l'aide de composés de type séléno-hydroxyacides, et plus particulièrement à l'aide d'acide 2-hydroxy-4-méthylsélénobutanoïque, sous forme (D,L), ou d'un énantiomère, sel, dérivé ester ou amide de ce composé, ainsi que l'utilisation des microorganismes photosynthétiques ainsi enrichis en nutrition animale ou humaine, cosmétique ou pharmacie.

Le sélénium est un micronutriment essentiel pour l'Homme et les mammifères notamment (Wendel, A. ; Phosphorus, Sulfur Silicon Relat Elem., 1992, 67, 1-4, 405-415). En particulier, il participe, sous la forme de L(+)-sélénocystéine ou de L(+)-sélénométhionine (Muller, S. et al., Arch. Microbiol., 1997, 168, 421) à la biosynthèse des sélénoprotéines telles que la Glutathion peroxydase, la Thiorédoxine réductase et la Sélénoprotéine P.

Chez l'Homme, des déficiences en sélénium ont été rapportées, notamment dans le cas de patients soumis à une alimentation par voie parentérale durant de longues périodes (Von Stockhausen, H.B., Biol. Trace Elem. Res., 1988, 15 :147-155). Une complémentation journalière de 200µg en sélénium est considérée comme sûre et adéquate pour un homme adulte de poids moyen (Schrauzer, G.N., J. Am. Col. Nutr., 2001, 20 :1-14).

Le sélenium se trouve dans la nature, sous deux formes : organique et inorganique.

Les composés inorganiques sont le plus souvent des sels tels que le sélénite ou le sélénate de sodium. Ces composés sont très toxiques pour l'homme et la plupart des animaux.

Les composés organiques (composés organoséléniés) sont représentés dans les organismes vivants notamment par les acides aminés L(+)-sélénométhionine, L(+)-méthylsélénocystéine et L(+)-sélénocystéine.

La L(+)-sélénométhionine est la principale source de sélénium organique chez l'Homme et les animaux. Cependant, l'Homme et les animaux sont autoxotrophes pour cet acide aminé, qui ne peut être obtenu qu'au travers de l'alimentation.

C'est donc sous cette forme organique qu'idéalement le sélénium devrait être incorporé dans les compléments alimentaires visant à traiter ou prévenir une carence en sélenium.

Il a pu être ainsi démontré qu'une supplémentation de l'alimentation en L(+)-sélénométhionine était beaucoup moins toxique et présentait une meilleure biodisponibilité qu'un apport sous forme de sélénite de sodium (Mony, M.C. et al., J. of Trace Elem. Exp. Med., 2000, 13 : 367-380).

Actuellement, on ne connaît pas d'autres voies métaboliques de captation du sélénium par les organismes vivants que celles utilisant comme substrats le sélénium inorganique, principalement sous la forme de sélénite de sodium, et la sélénométhionine.

On peut trouver un apport convenable en sélénium organique chez les végétaux supérieurs (blé, maïs, soja notamment), chez lesquels plus de 80% du sélénium est constitué par de la L(+)-sélénométhionine (Schrauzer, G.N., J.Am.Coll. Nutrit., 2001, 20 (1) :1-4). Cependant la concentration en sélénium de ces végétaux n'est pas suffisante pour pouvoir réaliser facilement, et à moindre coût, des additifs alimentaires.

Une des voies explorées pour obtenir des compositions riches en sélénométhionine, consiste à enrichir certains microorganismes en sélénium organique à partir de sélénium inorganique. Ces microorganismes, une fois enrichis, peuvent servir de matière première à la préparation de produits alimentaires ou cosmétiques.

De nombreuses publications décrivent, par exemple, la préparation de levures enrichies en sélénium, et plus particulièrement la levure *Saccharomyces cerevisiae* (Oh Tae-Kwang et al., brevet KR950006950 du 26.06.1995) en vue de les utiliser en tant que tels ou de les incorporer dans des compositions alimentaires (Moesgaard S. et al., brevet DK200200408 du 16.09.2003) ; ou encore d'obtenir des produits dérivés enrichis en sélénium tels que par exemple du pain (Wang Boaquan, brevet CN 1817143 du 16.08.2006), du lait (Jeng Chang-Yi, brevet TW565432 du 11.12.2003), des oeufs (Cui Li et al., brevet CN1302723C du 07.03.2007), du chocolat (In Gyeong Suk et al., brevet KR20040101145 du 08.11.2004) ou de la bière (Jakovleva L.G. et al., brevet RU2209237 du 27.07.2003) enrichis en sélénium. Dans le domaine des aliments de santé, des préparations contenant des levures enrichies en sélénium ont aussi été proposées pour des femmes enceintes (Wang Weiyi, brevet CN1778199 du 31.05.2006), ou encore pour améliorer le micro-environnement intestinal de patients hypoglycémiques (Li Tao Zhao, brevet CN1810161 du 02.08.2006). Dans le domaine dermocosmétique, des compositions contenant des levures enrichies en sélénium ont été développées en vue de réduire la perte capillaire (Kasik Heinz, brevet DE19858670 du 21.06.2000) ou en prévention du photo-vieillissement (Kawai Norihisa et al., brevet JP07300409 du 14.11.1995). Des préparations pharmaceutiques contenant des levures enrichies en sélénium ont été utilisées dans la prévention et le traitement de pathologies inflammatoires telles que les rétinopathies liées au diabète (Crary Ely J., brevet US5639482 du 17.06.1997), ou cardiovasculaires (Nagy P.L. et al.; brevet HUT060436 du 28.09.1992).

Les bactéries et plus particulièrement les bactéries pro-biotiques ont, elles aussi, fait l'objet d'enrichissement en sélénium (Calomme M. et al., Biol. Trace Elem. Res.,1995, 47, 379-383). *Lactobacillus acidophilus,* mais aussi *Lactobacillus reuteri, Lactobacillus ferintoshensis, Lactobacillus buchneril parabuchneri* (Andreoni V. et al., brevet US0258964) ont été décrites en tant que compléments alimentaires enrichis en sélénium. Des mélanges de pro-biotiques constitués de levures et de lactobacilles, en vue de renforcer le système immunitaire et la résistance aux maladies (Huang Kehe Qin, brevet CN1283171C du 08.11.2006) ont été préparés.

Cependant, dans toutes ces préparations, les microorganismes enrichis en sélénium sont préparés à partir de sélénium inorganique uniquement. Ainsi, la source de sélénium la plus souvent utilisée consiste en du sélénite ou du sélénate de sodium solubilisé dans les milieux de cultures des microorganismes. Les micro-organismes ainsi enrichis, bien qu'ayant synthétisé des quantités satisfaisantes de sélénium organique assimilable par l'organisme humain, présentent souvent un taux résiduel élevé en sélénium inorganique non transformé, ce qui peut s'avérer dangereux pour le consommateur.

Dans une précédente demande publiée sous WO 2006/008190, de nouveaux composés organiques de type séléno-hydroxyacides ont été décrits comme pouvant servir de précurseurs à la synthèse de L(+)-sélénométhionine chez l'homme et l'animal.

De manière surprenante, le demandeur a constaté que les composés organiques de type séléno-hydroxyacide tels que ceux décrits dans la demande WO 2006/008190 pouvaient être incorporés dans des milieux de culture pour enrichir différents microorganismes photosynthétiques en sélénium organique. Les résultats obtenus ont révélé que ces composés permettent d'enrichir très efficacement de tels microorganismes, notamment en L(+)-sélénométhionine, avec un rendement équivalent, voire supérieur, à celui obtenu à l'aide des composés inorganiques habituellement utilisés.

Il est ainsi apparu que l'enrichissement de microorganismes photosynthétiques en sélénium organique à partir de composés organiques de type séléno-hydroxyacide permettait de produire du sélénium organique exempt de sélénium inorganique, et ainsi de résoudre les problèmes de toxicité liés aux procédés de l'art antérieur.

Les microorganismes photosynthétiques ainsi enrichis peuvent être directement utilisés dans l'alimentation dans le cadre de la prévention ou du traitement des carences en sélénium, notamment en vue de produire des produits et compositions pharmaceutiques, nutritionnelles ou cosmétiques.

### Description détaillée de l'invention

La présente demande vise l'obtention de microorganismes photosynthétiques, c'est-à-dire de microorganismes dont la croissance est dépendante d'une source d'énergie lumineuse.

Par microorganisme, on entend tout organisme vivant unicellulaire appartenant à l'un des Règnes suivants : monères, protistes, mycètes ou protozoaires, présentant une structure cellulaire eucaryote ou procaryote, de taille microscopique ou ultramicroscopique, ayant un potentiel métabolique et de reproduction. Lesdits organismes unicellulaires peuvent être impliqués dans la formation de filaments ou de biofilms.

De préférence, les microorganismes photosynthétiques selon l'invention sont des micro-algues eucaryotes, ou des micro-algues procaryotes comme les cyanobactéries, préférentiellement des micro-algues du genre *Chlorella* ou du genre *Spirulina* ou *Arthrospira* (spiruline). Ces dernières sont bien connues de l'homme du métier pour être utilisées en tant que compléments alimentaires, notamment dans les pays en voie de développement.

Par sélénium organique, on entend un ensemble de molécules contenant au moins un composé possédant au moins un atome de sélénium dans sa structure chimique susceptible d'être produit par un organisme vivant, tels que notamment les acides aminés sélénométhionine, méthylsélénocystéine et sélénocystéine, des peptides ou des protéines les contenant.

Les microorganismes photosynthétiques ainsi enrichis en sélénium peuvent être utilisés en tant que tels, ou bien en tant qu'additif alimentaire. Ils peuvent, par exemple, être déshydratés pour former une poudre stable pouvant être incorporée dans des compositions servant de base à la préparation de produits transformés, mais peuvent être aussi utilisés vivants en tant que pro-biotiques dans les processus de transformation de produits alimentaires, en vue d'obtenir, par exemple, des laits ou boissons fermentés.

La présente invention a donc pour objet un nouveau procédé d'enrichissement de microorganisme photosynthétique en sélénométhionine et/ou en sélénocystéine, caractérisé en ce que ledit microorganisme photosynthétique est cultivé dans un milieu de culture comprenant un composé de type séléno-hydroxyacide de formule (I) selon la revendication 1, ou bien un sel, un dérivé ester ou amide dudit composé.

Un composé de type séléno-hydroxyacide est un composé de formule générale (I), un précurseur, un sel, ou bien un dérivé ester ou amide de celui-ci : 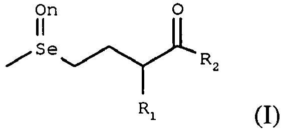 formule dans laquelle :
**n** est égal à 0, 1 ou 2 ;
**R₁** est un groupe OH, OCOR₃, OPO₃H₂, OPO₃R₄R₅ ou OR₆ ;
**R₂** est une groupe OH, R₃, NHR₇, S-cystéinyle ou S-glutathionyle ; étant entendu que lorsque n = 1 et R₂ est OH, alors R₁ ne peut être OH ;
**R₃** est un groupe alkoxyle, céramide 1, céramide 2, céramide 3, céramide 4, céramide 5, céramide 6a et 6b, S-cystéinyle, S-glutathionyle, ou un groupe choisi parmi les suivants :

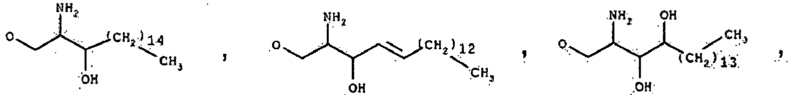

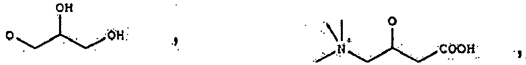

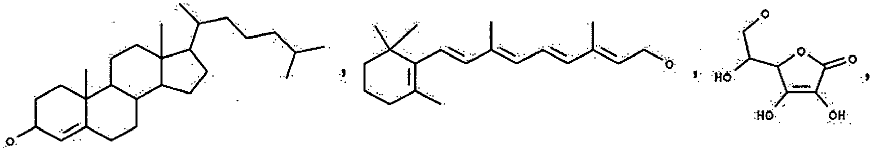

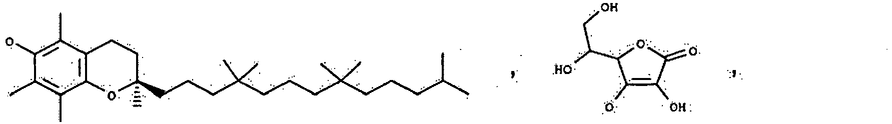

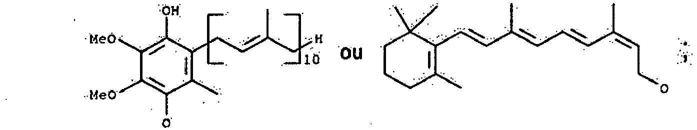

De préférence, **R₃** est un groupe alkoxyle, S-cystéinyle, S-glutathionyle;
**OR₄** est un groupe alkoxyle (C₁-C₂₆), céramide 1, céramide 2, céramide 3, céramide 4, céramide 5, céramide 6a et 6b, ou un groupe choisi parmi les suivants :

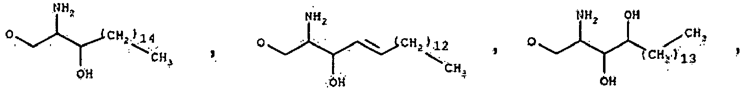

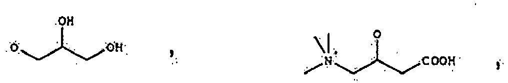

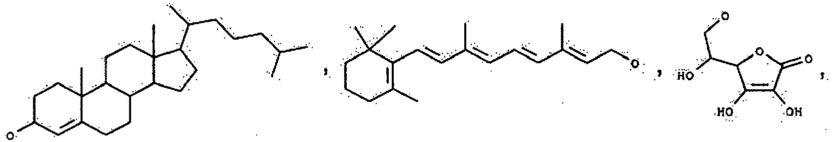

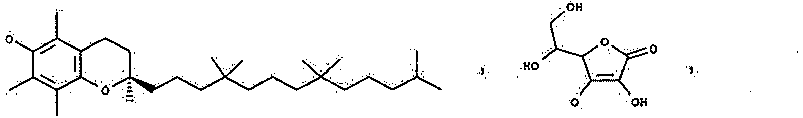

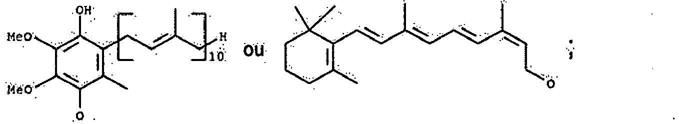

De préférence **OR₄** est un groupe alkoxyle (C₁-C₂₆);
**OR₅** est un groupe alkoxyle (C₁-C₂₆), céramide 1, céramide 2, céramide 3, céramide 4, céramide 5, céramide 6a et 6b ou un groupe choisi parmi les groupements suivants :

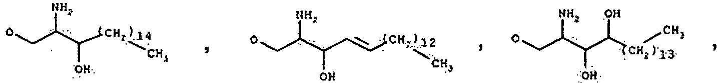

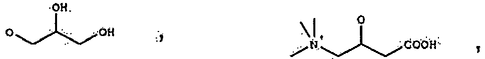

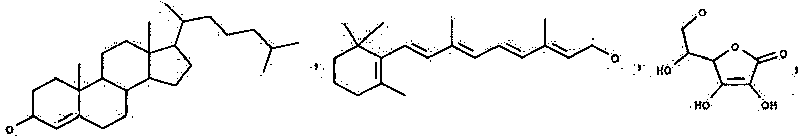

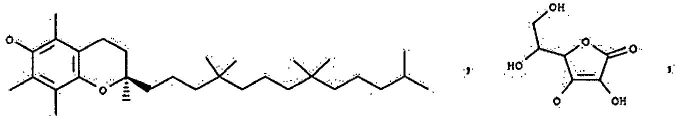

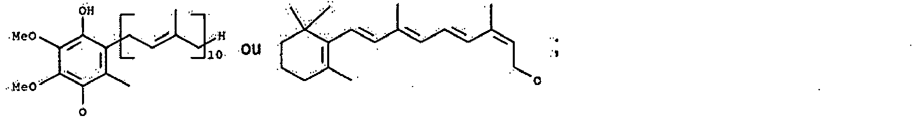

De préférence **OR₅** est un groupe alkoxyle (C₁-C₂₆).

**OR₆** est un groupe pyruvate, lactate, citrate, fumarate, maléate, myristate, palmitate, stéarate, palmitoléate, oléate, linoléate, acides gras naturels ou 13-cis rétinoate ;
**R₇** est un groupe H, alkyle (C₁-C₂₆), un amino-acide naturel ou une amine naturelle.

Dans la formule (I) ci-dessus :
- par alkyle, il est entendu un groupement comportant 1 à 26 atomes de carbone linéaire ou cyclique, éventuellement ramifié, éventuellement fluoré ou polyfluoré, et comportant éventuellement une ou plusieurs doubles liaisons carbone-carbone, tel que par exemple méthyle, éthyle, isopropyle, trifluorométhyle, linoléyle, linolényle, palmitoyle.
- par alkoxyle, il est entendu un groupement dérivé d'un alcool primaire, secondaire ou tertiaire comportant 1 à 26 atomes de carbone linéaire ou cyclique, éventuellement ramifié, éventuellement fluoré ou polyfluoré, et comportant éventuellement une ou plusieurs doubles liaisons carbone-carbone, tel que par exemple méthoxyle, éthoxyle, isopropoxyle, trifluorométhoxyle, linoléoxyle, linolénoxyle, palmitoxyle.

- des structures de radicaux de type céramide sont décrites notamment dans « Cosmetic Lipids and the Skin Barrier », Thomas Fôrster Ed. 2002, Marcel Dekker, Inc., p 2, fig 2.
- par naturel, il est entendu tout composé correspondant retrouvé dans le métabolisme des organismes du monde végétal, animal, ainsi que dans celui de l'homme (Steglich W., Römpp Encyclopedia Natural Products, G. Thieme ed.).
- par oligomère, il est entendu tout composé constitué par l'enchaînement de 2 à 15 monomères reliés entre eux par l'intermédiaire d'une liaison de type ester.
- par polymère, il est entendu tout composé constitué par l'enchaînement de plus de 15 monomères reliés entre eux par l'intermédiaire d'une liaison de type ester.

Le composé séléno-hydroxy-acide de formule (I) selon l'invention est celui de la revendication 1, pour lequel n = 0 et chacun de R1 et R2 est un hydroxyle, ou bien un sel, un dérivé ester ou amide dudit composé.

Selon l'invention, ledit composé de formule (I) est préférentiellement utilisé sous forme de sels de calcium, de zinc ou de magnésium, ce qui permet généralement une meilleure solubilité dans les milieux de culture, ainsi qu'une meilleure assimilation par les microorganismes photosynthétiques.

Dans un mode de réalisation préférée de l'invention, le microorganisme photosynthétique est choisi dans le groupe formé par les Cyanophycées et les Chlorophycées. Ainsi le microorganisme photosynthétique est avantageusement sélectionné parmi les Cyanophycées ou les Chlorophycées, de préférence choisi dans le groupe formé par les Chlorophycées et les Cyanophycées du genre *Spirulina* ou *Arthrospira.*

L'invention vise plus particulièrement l'utilisation d'un composé de formule (I) choisi (ou pris) parmi :
- l'acide L-2-hydroxy-4-méthylsélénobutanoïque,
- l'acide D-2-hydroxy-4-méthylsélénobutanoïque,
- l'acide DL-2-hydroxy-4- méthylsélénobutanoïque,
   ou un sel de ces composés.

Ces composés sont décrits dans la demande WO 2006/008190.

L'invention a également pour objet un microorganisme photosynthétique enrichi en sélénium organique, susceptible d'être obtenu selon le procédé de l'invention selon la revendication 7. Un tel microorganisme présente généralement un contenu en sélénium organique supérieur à 1000 ppm, plus préférentiellement supérieur à 2000 ppm en équivalent sélénium, et de préférence moins de 1,5 %, préférentiellement moins de 0,5 %, plus préférentiellement moins de 0,1 % en masse de sélénium inorganique par rapport au sélénium total.

Autrement dit, les résidus de sélénium sous forme inorganique présents dans les microorganismes photosynthétiques enrichis selon le procédé de l'invention, comptent généralement pour moins de 1,5 % du sélénium total présent dans les microorganismes, ce qui représente généralement moins de 0,5% de la biomasse sèche totale (poids sec) dudit microorganisme.

L'invention vise tout particulièrement un microorganisme photosynthétique enrichi en sélénium organique selon l'une des revendications 7 à 17. De préférence, il est caractérisé en ce que la teneur dudit microorganisme en sélénium sous forme de sélénométhionine représente plus de 70%, plus préférentiellement plus de 80% et encore plus préférentiellement plus de 90%, en masse de sélénium, par rapport au sélénium total présent dans ledit microorganisme photosynthétique. Une telle proportion en sélénométhionine représente une amélioration, en termes de quantité et en qualité de sélénium organique présent dans le microorganisme, importante et particulièrement avantageuse par rapport à ce qui était obtenu dans l'art antérieur.

En particulier, l'invention vise le cas où le microorganisme est caractérisé en ce qu'il est une micro-algue Chlorophycée enrichie en sélénium, et en ce que ledit microorganisme comprend une teneur en sélénométhionine généralement supérieure à 1000 µgSe/g en poids sec dudit microorganisme.

On exprime la quantité de sélénium fixée à l'intérieur des microorganismes sous forme de molécules organiques (sélénométhionine, sélénocyctéine ou autre) ou inorganiques (sels de sélénium) en masse de sélénium par gramme (µgSe/g) de poids sec des microorganismes. Autrement dit, la teneur en sélénium des microorganismes photosynthétiques est établie en calculant la masse de sélénium présente dans ces molécules, organiques ou inorganiques, rapportée à la biomasse sèche totale du microorganisme. De plus, les proportions en masse de sélénium présent sous forme organique et inorganique sont également établies et exprimées en pourcentages par rapport à la masse de sélénium total.

La teneur en sélénium total et sous forme de sélénométhionine des microorganismes photosynthétiques selon l'invention peut être déterminée respectivement par minéralisation et digestion enzymatique après centrifugation et lyophilisation des microorganismes, par exemple en suivant la méthode selon Lobinsky et *al*. décrite dans Mester, Z. et al. (2006) Annal. Bioanal. Chem. 385 :168-180.

Les résultats obtenus selon la présente invention, illustrés dans les exemples de la présente demande, montrent que les microorganismes photosynthétiques, plus particulièrement les micro-algues Chlorophycées et Cyanophycées accumulent du sélénium sous forme sélénométhionine en une teneur généralement supérieure à 1000 µgSe/g en poids sec et même supérieure à 1400 µgSe/g en poids sec de ces micro-algues.

L'invention vise donc plus particulièrement une Chlorophycée ou une Cyanophycée enrichie en sélénium organique, caractérisée en ce que sa teneur en sélénium organique sous forme de sélénométhionine, est généralement supérieure à 1000 µgSe/g en poids sec.

Une telle Chlorophycée ou cyanophycée enrichie en sélénium organique est généralement caractérisée en ce que sa teneur en sélénium organique sous forme de sélénométhionine, représente plus de 70%, plus préférentiellement plus de 80%, encore plus préférentiellement plus de 90%, en même plus de 95 % du sélénium total qu'elle contient, et également, en ce que sa teneur résiduelle en sélénium inorganique, généralement inférieure à 1,5 %, est préférentiellement inférieure à 0,5 %, plus préférentiellement inférieure à 0,1 % du sélénium total qu'elle contient. En général, sa teneur résiduelle en sélénium inorganique est inférieure à 1 %, préférentiellement inférieure à 0,5 %, plus préférentiellement inférieure à 0,2 %, et encore plus préférentiellement inférieure à 0,1% de la biomasse totale de ladite chlorophycée en poids sec.

L'invention vise en outre la fabrication de produits alimentaires, cosmétiques ou pharmaceutiques à partir desdits microorganismes photosynthétiques enrichis en sélénium suivant le procédé de la présente invention. Cette fabrication fait appel aux techniques connues de l'homme du métier.

Les microorganismes photosynthétiques selon l'invention peuvent également être utiles en nutrition animale, notamment en vue de l'obtention de dérivés secondaires enrichis en sélénium organique, comme par exemple du poisson, du lait ou des oeufs.

Les molécules et produits dérivés ainsi obtenus sont utiles dans différentes applications, dont celles rappelées en préambule, notamment en tant qu'agent cosmétique, pharmaceutique ou nutritionnel.

L'invention a également pour objet l'utilisation d'un microorganisme photosynthétique enrichi en sélénium selon l'invention des revendications 7 à 17 en tant que produit (ou agent) cosmétique, pharmaceutique (ou thérapeutique), ou nutritionnel.

L'invention vise également les compositions, généralement cosmétiques, pharmaceutiques ou nutritionnelles, comprenant lesdits microorganismes photosynthétiques.

L'invention vise encore un milieu de culture pour microorganisme photosynthétique, caractérisé en ce qu'il comprend le composé de formule (I) selon l'invention défini plus haut et selon la revendication 1. Un tel milieu de culture est utile pour la mise en oeuvre du procédé d'enrichissement des microorganismes photosynthétiques en sélénium selon l'invention.

En particulier, l'invention vise un milieu de culture solide ou liquide comprenant au moins un composé de formule (I), de préférence l'acide 2-hydroxy-4-méthylsélénobutanoïque ou l'un de ses sels, à une concentration comprise entre 0,5 et 2000 mg/L, de préférence entre 1 et 1000 mg/L, plus préférentiellement entre 2 et 500 mg/L, soit respectivement environ entre 0,2 et 800 mg/L dudit composé en équivalent sélénium, préférentiellement entre 0,4 et 400 mg/L dudit composé en équivalent sélénium, plus préférentiellement entre 0,8 et 200 mg/L dudit composé en équivalent sélénium.

Pour les micro-algues d'origine marine, les composés de formule (I) peuvent être dilués dans de l'eau de mer filtrée stérile ou dans de l'eau de mer synthétique fabriquée, par exemple, à partir du milieu « Reef Crystal » de la Société Aquarium Systems Inc., pour former un milieu minimum de culture.

Un procédé de préparation de micro-algues selon l'invention peut notamment comprendre une ou plusieurs des étapes suivantes :
- préparer un milieu de culture, de préférence un milieu minimum, comportant les éléments chimiques nécessaires à la croissance d'une micro-algue;
- introduire dans le milieu de culture un composé de formule (I), préférentiellement de l'acide 2-hydroxy-4méthylséléno-butanoïque en tant que source organique de sélénium ;
- ajuster le pH du mélange à une valeur comprise entre 6 et 10 ;
- mettre en culture un inoculum de préculture de ladite micro-algue dans le mélange ainsi constitué à une température comprise entre 12 et 45°C, sous une agitation orbitale comprise entre 100 et 500 rpm, et une atmosphère pouvant contenir de 0 à 20% en oxygène et de 0,3 à 20% en gaz carbonique, de préférence pendant 24 à 120 heures ;
- centrifuger le mélange entre 4000 et 10 000 rpm pendant quelques minutes, ou filtrer le mélange sur filtre 0,2 micromètre et rincer à l'eau physiologique sur le filtre;
- reprendre le culot cellulaire dans de l'eau physiologique ;
- centrifuger à nouveau entre 4000 et 10 000 rpm pendant quelques minutes ;
- récupérer le culot cellulaire humide dans lequel se trouvent les microalgues enrichies en sélénium ;
Le culot cellulaire humide peut être lyophilisé ou séché à l'air.

D'autres caractéristiques et avantages de l'invention sont donnés dans les exemples qui suivent. Les exemples ci-après sont fournis uniquement à titre d'illustration et ne sauraient en aucune façon limiter la portée de l'invention.

### EXEMPLES

### Exemple 1 : Production de la micro-algue Chlorella vulgaris enrichie en sélénium dans un milieu contenant de l'acide 2-hydroxy-4-méthylsélénobutanoïque (THD-177) en autotrophie

### Conditions expérimentales

La souche utilisée en photoautotrophie est *Chlorella vulgaris* SAG211-11 B : une souche axénique originaire de la collection SAG de l'Université de Göttingen (SAG : Sammlung von Algenkulturen der Universität Göttingen).

Cette souche a été cultivée dans le milieu BG -11 (Blue-Green medium) décrit par [Stanier RY et al. 1971 Bacteriol. Rev. 35 :171-205] dont la composition est la suivante (pour 1L) :
(1) NaNO₃ : 1,5g
(2) K₂HPO₄ : 0,04 g
(3) MgSO₄.7H₂O : 0,075 g
(4) CaCl₂. 2H₂O : 0,036 g
(5) Acide citrique : 0,006 g
(6) Citrate d'ammonium ferrique: 0,006g
(7) EDTA-Na₂ : 0,001 g
(8) Na₂CO₃: 0,02 g
(9) Eau distillée 1.0 L
(10) Solution d'éléments trace: 1mL / L
   H₃BO₃ : 2,86 g
   MnCl₂. 4H2O : 1,81 g
   ZnSO₄.7H₂O : 0,222 g
   Na₂MoO₄.2H₂: 0,39 g
   CuSO₄. 5H₂O : 0,08 g
   Co(NO₃)2. 6H₂O : 0,05 g

Le pH a été ajusté à 7,1 et le milieu a été autoclavé à 121°C pendant 15 minutes.

Cette souche a été cultivée à 25°C, 2400 +/-200 Lux en photoautrophie pendant 2 à 7 jours sous agitation orbitale (80 rpm), DO ᵢₙₜᵢₜ₆₆₀ₙₘ=0,05. La DO₆₆₀ₙₘ de la souche atteint 0,5 en 48h.

### Conditions de culture

La source organique de sélénium, à savoir l'acide 2-hydroxy-4-méthylséléno-butanoïque (THD-177, Tetrahedron SAS, France, CAS :873660-49-2) a été administrée à une concentration comprise entre 0,5 mg/L et 100 mg/L en équivalent sélénium, soit respectivement 1,25 mg/L et 250 mg/L d'acide 2-hydroxy-4-méthylsélénobutanoïque. L'addition du composé sélénié a été effectuée en une seule fois (soit pour 100 ml de culture une quantité comprise entre 0,125 mg et 25 mg) au démarrage de la culture ou en plusieurs fois à intervalles réguliers de temps, intervalles dont la durée a été comprise entre 6 et 24 heures, la culture ayant été maintenue pendant une durée de 2 à 7 jours.

### Exemple 2 : Production de la micro-algue Chlorella vulgaris enrichie en sélénium dans un milieu contenant de l'acide 2-hydroxy-4-méthylsélénobutanoïque (THD-177) (exemple selon l'invention) ou dans un milieu contenant du sélénite de sodium (exemple comparatif) en mixotrophie (présence de lumière et de carbohydrate - glucose - dans le milieu) :

Dans ces essais, la souche utilisée est une souche de *Chlorella vulgaris* SAG211-11 B : une souche axénique originaire de la collection SAG de l'Université de Göttingen (SAG : Sammlung von Algenkulturen der Universität Göttingen) qui a été cultivée en mixotrophie dans le milieu suivant :

| | |
|---|---|
| Yeast extracts | 0,33 g |
| Beef extracts | 0,33 g |
| Tryptose | 0,66g |
| FeSO4 | 0,66 mg |
| Glucose | 3,3 g |
| Eau distillée | qsp 1,0 L |

Le pH a été ajusté à 7,2 et le milieu a été autoclavé à 121°C pendant 15 minutes.

La source organique de sélénium, à savoir l'acide 2-hydroxy-4-méthylséléno-butanoïque (THD-177, Tetrahedron SAS, France, CAS :873660-49-2) a été administrée à une concentration de 20 mg/L en équivalent sélénium, soit 50 mg/L d'acide 2-hydroxy-4-méthylsélénobutanoïque.

La source de sélénium inorganique (SeNa, sélénite de sodium) a été administrée à une concentration de 20 mg/L en équivalent sélénium, soit 43,9 mg/L de sélénite de sodium.

L'addition du composé sélénié a été effectuée en une seule fois en phase exponentielle de croissance de la souche *Chlorella vulgaris* (soit 3 jours après l'inoculation)

### Préparation des échantillons pour analyses :

Après 7 jours d'incubation, le milieu a été filtré sur membrane Nalgène de 0,2 micron (Ref a-PES, diamètre 90 mm), et le rétentat cellulaire a été rincé avec de l'eau physiologique. La masse cellulaire humide a été lyophilisée pour l'analyse des constituants séléniés (sélénium total, sélénométhionine et sélénite de sodium).

### Analyse des constituants séléniés de Chlorella vulgaris

Le sélénium total a été dosé par ICP couplée à une détection par masse, après minéralisation de l'échantillon. La spéciation du sélénium a été effectuée par chromatographie liquide haute performance couplée à une détection masse-masse, après digestion enzymatique de l'échantillon, selon la méthode décrite par Lobinsky et *al*. dans Mester, Z. et al. (2006) Annal. Bioanal. Chem. 385 :168-180.

### Résultats

Le tableau 1 ci-dessous indique les valeurs moyennes en équivalents sélénium obtenues en triplicate pour des temps d'incubation de 7 jours.

**Tableau 1**

| Analyse des composants séléniés de la micro-algue *Chlorella vulgaris* | | | |
|---|---|---|---|
| | Se total mgSe/kg biomasse | SeMethionine mgSe/kg biomasse | Se(IV) mgSe/kg Biomasse |
| Ajout THD177 20mgSe/L | 1293±23 | 1274±109 (98,5% du Se total) | 6±1 (0,4% du Se total) |
| Ajout SeNa 20 mgSe/L | 144±5 | 29±2 (20% du Se total) | 4,1±0,4 (2,7% du Se total) |

Les résultats obtenus ont montré, pour une même dose de Sélénium ajoutée sous forme de -THD177 ou-de SeNa , ici 20 mgSe/L, que :
- neuf fois plus de Se total a été détecté si l'ajout est effectué sous forme de THD177 que si l'ajout est effectué sous forme de SeNa ;
- le niveau de sélénium accumulé intra-cellulairement sous forme de sélénométhionine obtenu par un ajout de THD177 est 44 fois plus important que celui obtenu par un ajout sous forme de SeNa ;
- le taux de sélénium accumulé intra-cellulairement sous forme de sélénométhionine atteint quasiment 100% (98,5%) des formes séléniées intra-cellulaires si l'ajout est effectué sous forme de THD177 par rapport à un taux de 20% avec le SeNa ; et que :
- seulement 0,4% de Se(IV) dans le Sélénium total ont été détectés si l'ajout est du THD177, alors que 2,7% de Se (IV) ont été détectés dans le sélénium total en cas d'ajout de SeNa.

### Exemple 3 : Production de la micro-algue Arthrospira platensis enrichie en sélénium dans un milieu contenant de l'acide 2-hydroxy-4-méthylsélénobutanoïque (THD-177) (exemple selon l'invention) ou dans un milieu contenant du sélénite de sodium (exemple comparatif) en autotrophie :

Dans ces essais, la souche utilisée est une souche de *Arthrospira platensis* 3054-E0001.

La souche 3054-E0001 a été cultivée en autotrophie dans le milieu suivant :

| | |
|---|---|
| Yeast extracts | 0,33 g |
| Beef extracts | 0,33 g |
| Tryptose | 0,66g |
| FeS04 | 0,66 mg |
| Eau distillée | qsp 1,0 L |

Le pH a été ajusté à 7,2 et le milieu a été autoclavé à 121°C pendant 15 minutes.

La source organique de sélénium, à savoir l'acide 2-hydroxy-4-méthylséléno-butanoïque (THD-177, Tetrahedron SAS, Franche, CAS :873660-49-2) a été administrée à une concentration de 25 mg/L en équivalent sélénium, soit 62,5 mg/L d'acide 2-hydroxy-4-méthylsélénobutanoïque.

La source de sélénium inorganique (SeNa, sélénite de sodium) a été administrée à une concentration de 25 mg/L en équivalent sélénium, soit 54,4 mg/L de sélénite de sodium.

L'addition du composé sélénié a été effectuée en une seule fois juste après l'inoculation de la souche *Arthrospira platensis* (soit T = 0).

### Préparation des échantillons pour analyses :

Après 10 jours d'incubation, le culot cellulaire a été filtré sur membrane Nalgène de 0,2 micron, et le rétentat cellulaire a été rincé avec de l'eau physiologique. La masse cellulaire humide a été lyophilisée pour l'analyse des constituants séléniés (sélénium total, sélénométhionine et sélénite de sodium).

### Analyse des constituants séléniés de Arthrospira platensis

Le sélénium total a été dosé par ICP couplée à une détection par masse, après minéralisation de l'échantillon. La spéciation du sélénium a été effectuée par chromatographie liquide haute performance couplée à une détection masse-masse, après digestion enzymatique de l'échantillon, selon la méthode décrite par Lobinsky et *al*. dans Mester, Z. et al. (2006) Annal. Bioanal. Chem. 385 :168-180.

### Résultats

Le tableau 2 ci-dessous indique les valeurs moyennes en équivalents sélénium obtenues en triplicate pour des temps d'incubation de 10 jours.

**Tableau 2**

| Analyse des composants séléniés de la micro-algue *Arthrospira platensis* | | | |
|---|---|---|---|
| | Se total mgSe/kg biomasse | SeMethionine mgSe/kg biomasse | Se(IV) mgSe/kg biomasse |
| Ajout THD177 25 mgSe/L | 1431 ±68 | 1402±47 (98% du Se total) | 17,2±0,7 (1,2 % du Se total) |
| Ajout SeNa 25 mgSe/L | 177±2 | 13±3 (7% du Se total) | 5,1±0,3 (2,9% du Se total) |

Les résultats obtenus ont montré, pour une même dose de sélénium ajoutée sous forme de THD177 ou de SeNa ajoutée, ici 25 mgSe/L, que :
- huit fois plus de Se total a été détecté pour un ajout effectué sous forme de THD177 que pour un ajout effectué sous forme de SeNa ;
- le niveau de sélénium accumulé intra-cellulairement sous forme de sélénométhionine obtenu par un ajout de THD177 est 108 fois plus important que celui obtenu par un ajout sous forme de SeNa ;
- le taux de sélénium accumulé intra-cellulairement sous forme de sélénométhionine atteint 98% des formes séléniées intracellulaires si l'ajout est effectué sous forme de THD177 par rapport à un taux de 7% avec le SeNa ; et que :
- seulement 1,2% de Se(IV) dans le Sélénium total ont été détectés si l'ajout est du THD177, alors que 2,9% de Se (IV) ont été détectés dans le sélénium total en cas d'ajout de SeNa.

### Exemple 4 : Production de la micro-algue Arthrospira platensis enrichie en sélénium dans un milieu contenant de l'acide 2-hydroxy-4-méthylsélénobutanoïque (THD-177) (exemple selon l'invention)

Dans ces essais, la souche utilisée est une souche de *Arthrospira platensis* 3054-E0001. On compare les résultats
- de l'exemple précédent, l'exemple 3, au cours duquel l'addition selon l'invention du composé sélénié THD-177 a été effectuée en une seule fois juste après l'inoculation de la souche *Arthrospira platensis,* avec
- une nouvelle expérience où l'addition selon l'invention du composé sélénié THD-177 a été effectuée en phase exponentielle de culture de ladite souche *Arthrospira platensis,* comme dans l'exemple 2.

### Résultats

Le tableau 3 ci-dessous indique les valeurs moyennes en équivalents sélénium obtenues en triplicate pour des temps d'incubation de 10 jours.

**Tableau 3**

| Analyse des composants séléniés de la micro-algue *Arthrospira platensis* | | | | |
|---|---|---|---|---|
| Analyse des composants séléniés de la micro-algue *Arthrospira platensis* | Se total mgSe/kg biomasse | SeMethionine mgSe/kg biomasse | THD177 mgSe/kg biomasse | Se(IV) mgSe/kg biomasse |
| Ajout à l'inoculation THD177 25 mgSe/L | 1431±68 | 1402±47 (98% du Se total) | 5±1 (0,35 % du Se total) | 17,2±0,7 (1,2 % du Se total) |
| Ajout phase exponentielle THD177 25 mgSe/L | 1274±16 | 1078±89 (85 % du Se total) | 11±2 (0,86 % du Se total) | 14±2 (1,1 % du Se total) |

Les résultats ont montré que 12% de plus de sélénium total et 30% de plus de sélénium sous forme sélénométhionine ont été obtenus dans le test « ajout à T0 » par rapport au test « ajout en phase exponentielle ». Cette différence pourrait être la conséquence du temps de contact plus long de la biomasse avec le THD177 dans le test « ajout à T0 » que dans l'autre test « ajout en phase exponentielle ».

Dans les deux cas, le taux intracellulaire en Se (IV) demeure faible à 1% du sélénium total.

## Revendications

1. Procédé d'enrichissement d'un microorganisme photosynthétique en sélénium organique, **caractérisé en ce que** ledit microorganisme photosynthétique est cultivé dans un milieu comprenant un composé de type séléno-hydroxyacide de formule générale (I) telle que définie ci-après, un sel, un dérivé ester ou amide dudit composé.

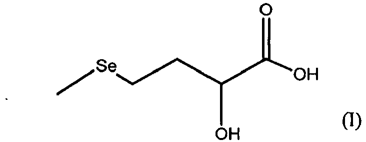

2. Procédé selon la revendication 1, dans lequel ledit microorganisme est enrichi en sélénométhionine.

3. Procédé selon l'une des revendications 1 ou 2, **caractérisé en ce que** ledit composé de formule (I) est choisi parmi :
- l'acide L-2-hydroxy-4-méthylsélénobutanoïque,
- l'acide D-2-hydroxy-4-méthylsélénobutanoïque,
- l'acide DL-2-hydroxy-4-méthylsélénobutanoïque,
ou un sel de ces composés.

4. Procédé selon l'une des revendications 1 à 3, **caractérisé en ce que** lesdits composés de formule (I) sont sous forme de sels de calcium, de zinc ou de magnésium.

5. Procédé selon l'une quelconque des revendications 1 à 4, **caractérisé en ce que** le microorganisme photosynthétique est choisi dans le groupe formé par les Cyanophycées, les Chlorophycées, et les *Chlorella.*

6. Procédé selon la revendication 5, **caractérisé en ce que** le microorganisme photosynthétique est choisi dans le groupe formé par les Cyanophycées du genre *Spirulina* ou *Arthrospira.*

7. Microorganisme photosynthétique enrichi en sélénium organique susceptible d'être obtenu selon le procédé de l'une quelconque des revendications 1 à 6, ledit microorganisme photosynthétique étant **caractérisé en ce que** sa teneur en sélénium organique, sous forme de sélénométhionine, est supérieure à 1000 µgSe/g en poids sec dudit microorganisme photosynthétique.

8. Microorganisme photosynthétique enrichi en sélénium organique selon la revendication 7, ledit microorganisme photosynthétique étant **caractérisé en ce que** sa teneur en sélénium organique, sous forme de sélénométhionine, représente plus de 70%, plus préférentiellement plus de 80% et encore plus préférentiellement plus de 90%, en masse de sélénium, par rapport au sélénium total présent dans ledit microorganisme photosynthétique.

9. Microorganisme photosynthétique enrichi en sélénium organique selon l'une quelconque des revendications 7 et 8, **caractérisé en ce que** le microorganisme photosynthétique comprend moins de 1,5 %, préférentiellement moins de 0,5 %, plus préférentiellement moins de 0,1 % en masse du sélénium inorganique par rapport au sélénium total.

10. Chlorophycée enrichie en sélénium organique selon l'une quelconque des revendications 7 à 9, **caractérisée en ce que** sa teneur en sélénium organique sous forme de sélénométhionine, est supérieure à 1000 µgSe/g en poids sec de ladite Chlorophycée.

11. Chlorophycée enrichie en sélénium organique selon la revendication 10, **caractérisée en ce que** sa teneur en sélénium organique sous forme de sélénométhionine, représente plus de 70%, plus préférentiellement plus de 80%, et encore plus préférentiellement plus de 90%, de la masse total de sélénium qu'elle contient.

12. Chlorophycée enrichie en sélénium organique selon l'une quelconque des revendications 10 et 11, **caractérisée en ce que** sa teneur résiduelle en sélénium inorganique est inférieure à 2 %, préférentiellement inférieure à 1,5%, plus préférentiellement inférieure à 1 % en masse, par rapport au sélénium total qu'elle contient.

13. Microorganisme photosynthétique enrichi en sélénium organique selon l'une des revendications 7 à 9, **caractérisé en ce qu'**il appartient au genre *Chlorella.*

14. Cyanophycée enrichie en sélénium organique selon l'une quelconque des revendications 7 à 9, **caractérisée en ce que** sa teneur en sélénium organique sous forme de sélénométhionine, est supérieure à 1000 µgSe/g en poids sec de ladite Cyanophycée.

15. Cyanophycée enrichie en sélénium organique selon la revendication 14, **caractérisée en ce que** sa teneur en sélénium organique sous forme de sélénométhionine, représente plus de 70%, plus préférentiellement plus de 80%, et encore plus préférentiellement plus de 90% de masse de sélénium total qu'elle contient.

16. Cyanophycée enrichie en sélénium organique selon l'une quelconque des revendications 14 et 15, **caractérisée en ce que** sa teneur résiduelle en sélénium inorganique est inférieure à 2 %, préférentiellement moins de 1,5 %, plus préférentiellement moins de 1 % en masse, par rapport au sélénium total qu'elle contient.

17. Cyanophycée selon l'une quelconque des revendications 14 à 16, **caractérisée en ce qu'**elle appartient au genre *Spirulina* ou *Arthrospira.*

18. Utilisation d'un microorganisme photosynthétique enrichi en sélénium organique selon l'une des revendications 7 à 17, en tant qu'agent cosmétique ou nutritionnel.

19. Composition comprenant au moins un microorganisme photosynthétique enrichi en sélénium organique selon l'une des revendications 7 à 17.

20. Composition selon la revendication 19, **caractérisée en ce que** ladite composition est cosmétique, pharmaceutique ou nutritionnelle.

21. Milieu de culture pour microorganisme photosynthétique, **caractérisé en ce qu'**il comprend un ou plusieurs composés de type séléno-hydroxyacide de formule (I) tel(s) que défini(s) dans la revendication 1.

## Patentansprüche

1. Verfahren zur Anreicherung eines photosynthetischen Mikroorganismus mit organischem Selen, **dadurch gekennzeichnet, dass** der photosynthetische Mikroorganismus in einem Medium kultiviert wird, das eine wie im Folgenden definierte Verbindung vom Selenohydroxysäure-Typ der allgemeinen Formel (I), ein Salz, ein Ester- oder Amidderivat der Verbindung umfasst:

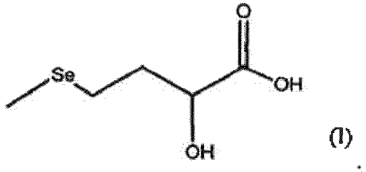

2. Verfahren nach Anspruch 1, wobei der Mikroorganismus mit Selenomethionin angereichert wird.

3. Verfahren nach einem der Ansprüche 1 oder 2, **dadurch gekennzeichnet, dass** die Verbindung der Formel (I) aus folgenden ausgewählt ist:
- L-2-Hydroxy-4-methylselenobuttersäure,
- D-2-Hydroxy-4-methylselenobuttersäure,
- DL-2-Hydroxy-4-methylselenobuttersäure
oder aus einem Salz aus diesen Verbindungen.

4. Verfahren nach einem der Ansprüche 1 bis 3, **dadurch gekennzeichnet, dass** die Verbindungen der Formel (I) in der Form von Calcium-, Zink- oder Magnesiumsalzen vorliegen.

5. Verfahren nach einem der Ansprüche 1 bis 4, **dadurch gekennzeichnet, dass** der photosynthetische Mikroorganismus aus der Gruppe ausgewählt wird, die von den Cyanophyceen, den Chlorophyceen und den *Chlorella* gebildet ist.

6. Verfahren nach Anspruch 5, **dadurch gekennzeichnet, dass** der photosynthetische Mikroorganismus aus der Gruppe ausgewählt wird, die von den Cyanophyceen der Gattung *Spirulina* oder *Arthrospira* gebildet ist.

7. Mit organischem Selen angereicherter photosynthetischer Mikroorganismus, der gemäß dem Verfahren nach einem der Ansprüche 1 bis 6 erhalten werden kann, wobei der photosynthetische Mikroorganismus **dadurch gekennzeichnet ist, dass** sein Gehalt an organischem Selen in der Form von Selenomethionin höher als 1000 µg Se/g des Trockengewichts des photosynthetischen Mikroorganismus ist.

8. Mit organischem Selen angereicherter photosynthetischer Mikroorganismus nach Anspruch 7, wobei der photosynthetische Mikroorganismus **dadurch gekennzeichnet ist, dass** sein Gehalt an organischem Selen in der Form von Selenomethionin mehr als 70 Massen-%, mehr bevorzugt mehr als 80 Massen-% und noch mehr bevorzugt mehr als 90 Massen-% der Selenmasse in Bezug auf das gesamte Selen, das in dem photosynthetischen Mikroorganismus vorliegt, ausmacht.

9. Mit organischem Selen angereicherter photosynthetischer Mikroorganismus nach einem der Ansprüche 7 und 8, **dadurch gekennzeichnet, dass** der photosynthetische Mikroorganismus weniger als 1,5 Massen-%, vorzugsweise weniger als 0,5 Massen-%, mehr bevorzugt weniger als 0,1 Massen-% anorganisches Selen in Bezug auf das gesamte Selen umfasst.

10. Mit organischem Selen angereicherte Chlorophycee nach einem der Ansprüche 7 bis 9, **dadurch gekennzeichnet, dass** ihr Gehalt an organischem Selen in der Form von Selenomethionin höher als 1000 µg Se/g des Trockengewichts der Chlorophycee ist.

11. Mit organischem Selen angereicherte Chlorophycee nach Anspruch 10, **dadurch gekennzeichnet ist, dass** ihr Gehalt an organischem Selen in der Form von Selenomethionin mehr als 70 Massen-%, mehr bevorzugt mehr als 80 Massen-% und noch mehr bevorzugt mehr als 90 Massen-% des gesamten Selens, das sie enthält, ausmacht.

12. Mit organischem Selen angereicherte Chlorophycee nach einem der Ansprüche 10 und 11, **dadurch gekennzeichnet ist, dass** ihr Restgehalt an anorganischem Selen kleiner als 2 Massen-%, vorzugsweise kleiner als 1,5 Massen-%, mehr bevorzugt kleiner als 1 Massen-% in Bezug auf das gesamte Selen, das sie enthält, ist.

13. Mit organischem Selen angereicherter photosynthetischer Mikroorganismus nach einem der Ansprüche 7 bis 9, **dadurch gekennzeichnet, dass** er zur Gattung *Chlorella* gehört.

14. Mit organischem Selen angereicherte Cyanophycee nach einem der Ansprüche 7 bis 9, **dadurch gekennzeichnet, dass** ihr Gehalt an organischem Selen in der Form von Selenomethionin höher als 1000 µg Se/g des Trockengewichts der Cyanophycee ist.

15. Mit organischem Selen angereicherte Cyanophycee nach Anspruch 14, **dadurch gekennzeichnet ist, dass** ihr Gehalt an organischem Selen in der Form von Selenomethionin mehr als 70 Massen-%, mehr bevorzugt mehr als 80 Massen-% und noch mehr bevorzugt mehr als 90 Massen-% des gesamten Selens, das sie enthält, ausmacht.

16. Mit organischem Selen angereicherte Cyanophycee nach einem der Ansprüche 14 und 15, **dadurch gekennzeichnet, dass** ihr Restgehalt an anorganischem Selen kleiner als 2 Massen-%, vorzugsweise kleiner als 1,5 Massen-%, mehr bevorzugt kleiner als 1 Massen-% in Bezug auf das gesamte Selen, das sie enthält, ist.

17. Cyanophycee nach einem der Ansprüche 14 bis 16, **dadurch gekennzeichnet, dass** sie zur Gattung *Spirulina* oder *Arthrospira* gehört.

18. Verwendung eines mit organischem Selen angereicherten photosynthetischen Mikroorganismus nach einem der Ansprüche 7 bis 17 als kosmetisches Mittel oder Nahrungsmittel.

19. Zusammensetzung, die mindestens einen mit organischem Selen angereicherten photosynthetischen Mikroorganismus nach einem der Ansprüche 7 bis 17 umfasst.

20. Zusammensetzung nach Anspruch 19, **dadurch gekennzeichnet, dass** die Zusammensetzung eine kosmetische, pharmazeutische oder Nahrungszusammensetzung ist.

21. Kulturmedium für einen photosynthetischen Mikroorganismus, **dadurch gekennzeichnet, dass** es eine oder mehrere wie in Anspruch 1 definierte Verbindungen vom Selenohydroxysäure-Typ der Formel (I) umfasst.

## Claims

1. Method for enriching a photosynthetic microorganism in organic selenium, **characterized in that** the photosynthetic microorganism is cultured in a medium comprising a compound of selenohydroxy acid type of general formula (I) as defined below, a salt or an ester or amide derivative of said compound:

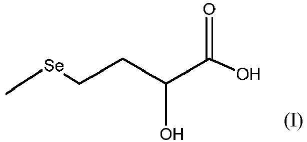

2. The method according to claim 1, wherein the microorganism enriched in organic selenium is enriched in selenomethionine.

3. The method according to claim 1 or 2, **characterized in that** the compound of formula (I) is chosen among:
- L-2-hydroxy-4-methylselenobutanoic acid,
- D-2-hydroxy-4-methylselenobutanoic acid, and
- DL-2-hydroxy-4-methylselenobutanoic acid,
or a salt of these compounds.

4. The method according to any one of claims 1 to 3, **characterized in that** the compounds of formula (I) are in the form of calcium, zinc or magnesium salts.

5. The method according to any one of claims 1 to 4, **characterized in that** the photosynthetic microorganism is selected from the group consisting of Cyanophyceae, Chlorophyceae and *Chlorella.*

6. The method according to claim 5, **characterized in that** the photosynthetic microorganism is selected from the group consisting of Cyanophyceae of the *Spirulina* or *Arthrospira* genus.

7. A photosynthetic microorganism enriched in organic selenium, obtainable according to the method of any one of claims 1 to 6, said photosynthetic microorganism being **characterized in that** its content of organic selenium in the form of selenomethionine is greater than 1000 µgSe/g by dry weight of said photosynthetic microorganism.

8. The photosynthetic microorganism enriched in organic selenium according to claim 7, said photosynthetic microorganism being **characterized in that** its content of organic selenium in the form of selenomethionine in said photosynthetic microorganism represents more than 70%, preferably more than 80%, and even more preferably more than 90%, by mass of selenium, relative to the total selenium present in said photosynthetic microorganism.

9. The photosynthetic microorganism enriched in organic selenium according to any one of claims 7 and 8, **characterized in that** the photosynthetic microorganism comprises less than 1.5%, preferably less than 0.5%, more preferably less than 0.1% by mass of inorganic selenium relative to the total selenium.

10. A Chlorophyceae enriched in organic selenium according to any one of claims 7 to 9, **characterized in that** its content of organic selenium in the form of selenomethionine is greater than 1000 µgSe/g by dry weight of said Chlorophyceae.

11. The Chlorophyceae enriched in organic selenium according to claim 10, **characterized in that** its content of organic selenium in the form of selenomethionine represents more than 70%, more preferably more than 80%, and even more preferably more than 90%, of the total mass of selenium it contains.

12. The Chlorophyceae enriched in organic selenium according to any one of claims 10 and 11, **characterized in that** its residual content of inorganic selenium is less than 2%, preferably less than 1.5%, more preferably less than 1% by mass, relative to the total selenium it contains.

13. The photosynthetic micro-organism according to any one of claims 7 to 9, **characterized in that** it belongs to the *Chlorella* genus.

14. A Cyanophyceae enriched in organic selenium according to any one of claims 7 to 9, **characterized in that** its content of organic selenium in the form of selenomethionine is greater than 1000 µgSe/g by dry weight of said Cyanophyceae.

15. The Cyanophyceae enriched in organic selenium according to claim 14, **characterized in that** its content of organic selenium in the form of selenomethionine represents more than 70%, more preferably more than 80%, and even more preferably more than 90% of the total mass of selenium it contains.

16. The Cyanophyceae enriched in organic selenium, according to any one of claims 14 and 15, **characterized in that** the residual content of inorganic selenium is less than 2%, preferably less than 1.5%, more preferably less than 1% by mass, relative to the total selenium it contains.

17. The Cyanophyceae according to any one of claims 14 to 16, **characterized in that** it belongs to the *Spirulina* or *Arthrospira* genus.

18. Use of a photosynthetic microorganism enriched in organic selenium according to any one of claims 7 to 17, as a cosmetic or nutritional agent.

19. A composition comprising at least one photosynthetic microorganism enriched in organic selenium according to any one of claims 7 to 17.

20. Composition according to claim 19, **characterized in that** said composition is a cosmetic, pharmaceutical or nutritional composition.

21. Culture medium for a photosynthetic micro-organism, wherein the culture medium comprises at least one compound of selenohydroxy acid type of formula (I), as defined in claim 1.
